# EUROPÄISCHE PATENTSCHRIFT

(11) **EP 1 948 286 B1**
(45) Veröffentlichungstag und Bekanntmachung des Hinweises auf die Patenterteilung: **07.12.2016**
(21) Anmeldenummer: 06804852.9
(22) Anmeldetag: 02.11.2006
(51) Int. Cl.: A61M 25/10

(54) **BALLONKATHETER**
BALLOON CATHETER
CATHETER A BALLON

(30) Priorität: 14.11.2005 CH 18192005
(43) Veröffentlichungstag der Anmeldung: 30.07.2008
(73) Patentinhaber: SIS Medical AG, 8500 Frauenfeld (CH)
(72) Erfinder: SCHWAGER, Michael, CH-8404 Winterthur (CH)
(74) Vertreter: Roshardt, Werner Alfred
(86) Internationale Anmeldenummer: PCT/CH2006/000618
(87) Internationale Veröffentlichungsnummer: WO 2007/053967

(56) Entgegenhaltungen:
- EP-A2- 0 266 957
- WO-A2-98/09670
- US-A- 5 358 487
- US-A- 5 447 497

## Beschreibung

Die Erfindung bezieht sich auf einen Ballonkatheter zur Einführung in ein im wesentlichen röhrenförmiges Hohlorgan im menschlichen oder tierischen Körper mit wenigstens einem befüll- und entleerbaren Ausdehnungsraum zwischen einer Vorder- und einer Rückseite, welchem Ausdehnungraum über wenigstens ein Anschlussstück mit wenigstens einem Lumen ein flüssiges oder gasförmiges Medium zu- und abführbar ist, und einem Führungsdrahtlumen mit einem flexiblen Führungsdraht entlang einer Ballonkatheter-Längsachse. Weiter betrifft die Erfindung ein Verfahren zum Betrieb des Ballonkatheters.

Ein solcher Ballonkatheter wird in WO9809670 beschrieben.

Die wichtigsten Unterscheidungskriterien von bekannten Katheterarten beziehungsweise -typen können beispielsweise folgendermassen zusammengefasst werden:
- Anwendungsart: Mensch oder Tier, diagnostisch (untersuchungsbedingt) oder therapeutisch (behandlungsbedingt).
- Betroffenes Hohlorgan: Harnröhre, Speiseröhre, Gallengänge, Gefässe, Adern, insbesondere Herzarterien.
- Verwendetes Material: Metalle, Legierungen, Glas, Latex, Silikon, Gummi, Kunststoffe, insbesondere PVC, mit einer Wandstärke von etwa 20 µm.
- Materialeigenschaften: Flexibilität, Festigkeit, Gleitfähigkeit.
- Nutzungshäufigkeit: Einmalige oder mehrmalige Nutzung.
- Einsatzdauer: Kurzzeit- oder Langzeitanwendung.
- Fixierung: Selbst fixierend, insbesondere mittels eines Ballons, oder nicht selbst fixierend.
- Geschlechtsspezifische Bauform: Für weibliches, männliches oder für beide Geschlechter geeignet.
- Bauform im Allgemeinen: Einkanal-/Mehrkanalkatheter, Anzahl und Anordnung der Öffnungen; gerade/gebogen, mit/ohne Erweiterung, unbeschichtet/beschichtet, mit/ohne Wandverstärkung, Einführung und Anwendung ohne/mit Hilfsmitteln, z. B. Spreiz- und/oder Gleitmitteln etc.

Mindestens ein Ausdehnungsraum des Ballonkatheters wird mit einem flüssigen oder mit einem gasförmigen Medium gefüllt und entleert. Ein Ballonkatheter dient dazu, einen bestimmten Kathetertyp im betreffenden Hohlorgan eines Menschen oder eines Tieres zu fixieren. Dabei ist es wichtig, den Ballonkatheter bezüglich der Wandstärke möglichst klein zu bauen. Gleichzeitig sollen die Gleit- und Flexibilitätseigenschaften möglichst gut und die Reissgefahr während des Einführvorgangs klein sein.

Der Ballonkatheter muss nach Einfüllen beziehungsweise nach dem Hineinpumpen des flüssigen oder gasförmigen Mediums einem möglichst grossen Arbeitsdruck von 10 bis 30 bar sicher standhalten. Dies, um einerseits das betreffende Hohlorgan beim Erreichen des Arbeitsdrucks im gewünschten Ausmass zu weiten und so Platz für die entsprechende Diagnose oder Behandlung zu schaffen. Andererseits aber auch, um ein unerwünschtes Ausweiten, zum Beispiel nach einer gewissen Zeit unter Arbeitsdruck, oder gar ein Platzen des Ballonkatheters, zu vermeiden. Ein unkontrolliertes Ausweiten des Hohlorgans könnte beispielsweise eine Arterie sprengen, ein Platzen des Ballonkatheters wäre überdies verbunden mit einem unkontrollierten, explosionsartigen Entweichen des eingefüllten Mediums in das Organ. Beides könnte zu einer lebensbedrohlichen Situation für Mensch oder Tier führen.

Der Erfinder hat sich die Aufgabe gestellt, einen Ballonkatheter der eingangs genannten Art zu schaffen, der
- bei gleicher Flexibilität eine erhöhte Druckfestigkeit hat,
- bei gleicher Druckfestigkeit eine erhöhte Flexibilität hat, oder
- gleichzeitig eine erhöhte Flexibilität und Druckfestigkeit aufweist.

Die Aufgabe wird erfindungsgemäss durch die Merkmale des Anspruchs 1 gelöst.

Überraschend hat sich gezeigt, dass der Arbeitsdruck eines Ballonkatheters im Verhältnis zur Wandstärke wesentlich erhöht werden kann, wenn zwei oder mehr gleitfähige Ballonhüllen direkt aufeinander liegen, auch wenn ihre Gesamtdicke wesentlich kleiner ist als diejenige eines Ballonkatheters mit einer einzelnen Ballonhülle (Single-Ballon). Gleichzeitig stützen sich die Ballonhüllen unter einem Arbeitsdruck gegenseitig. Die Gleitfähigkeit zwischen zwei Ballonflächen erlaubt eine Relativbewegung der Materialhüllen entlang ihrer Mantelflächen, was im Vergleich zum Single-Ballon auch eine grössere Flexibilität bei gleicher oder grösserer Gesamtdicke zulässt. Somit können sich die erfindungsgemässen Ballonhüllen dem spezifischen Verlauf eines röhrenförmigen Hohlorgans besser anpassen, sowohl während der Einführung als auch beim Befüllen/Entleeren des Ballonkatheters mit dem flüssigen oder gasförmigen Medium.

Es werden vorzugsweise Ballonmaterialien eingesetzt, die unter Berücksichtigung der Gleitfähigkeit aus einem flexiblen und zudem nur gering dehnbaren Werkstoff bestehen, vorzugsweise aus einem Polyamid, Nylon und/oder Polyester, wodurch der Arbeitsdruck nochmals gesteigert wird. Gleichzeitig wird eine Reissgefahr beim Einführen des Ballonkatheters in das entsprechende Hohlorgan minimiert.

Neben einer äusseren Gleitfähigkeit eines Ballonkatheters wird die Gleitfähigkeit zwischen zwei Ballonhüllen durch eine dünne Beschichtung von wenigstens einer der direkt aufeinander liegenden Ballonhüllen erhöht, vorzugsweise mit einem aufgetragenen Kunststoff oder einem Gleitfilm.

Messungen haben ergeben, dass ein Ballonkatheter in Doppelwandausführung mit einem maximalen Arbeitsdruck bis etwa 50 bar, vorzugsweise 40 bis 45 bar, sicher eingesetzt wird und sich bei höchstem Arbeitsdruck bezüglich des Durchmessers maximal nur wenige Bruchteile eines Millimeters weitet. Eine Doppelwandausführung bedeutet, dass zwei ballonförmige Hüllen ineinander geführt sind, welche direkt aufeinander liegen und gegeneinander gleitfähig sind.

Eine einfache Ballonhülle des erfindungsgemässen Ballonkatheters weist eine Wandstärke von nur etwa 3 µm auf. Drei gleitfähig ineinander gelegte Ballonhüllen sind in ihrer Gesamtwandstärke noch immer weniger als halb so dick wie ein üblicher Single-Ballon. Die Arbeitsdruckwerte der Doppelwandausführung lassen sich drei Ballonhüllen nochmals verbessern, sie liegen bei Werten bis etwa 60 bar, vorzugsweise 50 bis 55 bar. Die Dreifachballonhüllen weiten sich beim höchsten Arbeitsdruck im Durchmesser maximal nur etwa 0,2 mm.

Mit jeder weiteren Ballonhülle verkleinern sich die Dehnungswerte, jedoch verkleinert sich gleichzeitig die Flexibilität. Die relative Zunahme des Arbeitsdrucks nimmt mit jeder weiteren Ballonhülle ab, bis ein Grenzwert erreicht ist und/oder die Flexibilität ein gewünschtes Mass unterschreitet. In der Praxis liegt die Grenze meist bei einer Dreifachballonhülle.

Die wesentlichen Vorteile von direkt aufeinander liegenden Ballonhüllen können wie folgt zusammengefasst werden:
- Der erfindungsgemässe Ballonkatheter in Doppelwand- und Dreifachausführung hat bei geringerer totaler Wandstärke gegenüber Single-Ballonen gleichzeitig eine erhöhte Flexibilität und Druckfestigkeit.
- Gleitfähigkeit und Reissfestigkeit des Ballonkatheters in einem Hohlorgan bleiben im Vergleich zum Stand der Technik wenigstens erhalten.

Der Führungsdraht selbst mit sehr kleinem Durchmesser ragt über den Ballonkatheter hinaus und wird dank seines geringen Widerstands und der Flexibilität im Hohlorgan vorgeschoben. Der Ballonkatheter folgt dem Führungsdraht und schmiegt sich dem Verlauf des betreffenden Organs an. Der Führungsdraht besteht aus gut gleitfähigem, flexiblem und gleichzeitig zähem Material, vorzugsweise aus rostfreiem Stahl, Aluminium oder aus Kunststoffen mit hoher mechanischer Festigkeit, zum Beispiel PVC, Polyamid oder Polyester.

Der Führungsdraht ist gegenüber einem Ballon vorzugsweise abgedichtet. Diese Eigenschaft ist insbesondere dann von Vorteil, wenn die Erfindung nur einen einzigen Ausdehnungsraum aufweist. In der Herstellung wird wenigstens ein Arbeitsschritt gespart, eine potentielle Schwachstelle bleibt ausgeschlossen und je nach üblicherweise verwendetem Abdichtverfahren (z.B. Schweissen, Kleben, Ultraschall) wird eine nennenswerte Verdickung des Ballondurchmessers an der betreffenden Stelle vermieden.

Wenigstens eine Ballonhülle und/oder der Führungsdraht können gefärbt, reflektierend und/oder fluoreszierend ausgebildet sein, auch nur teilweise. Weiter kann im Inneren des Ballonkatheters wenigstens eine röntgendichte Markierung angebracht sein, beispielsweise in Form eines röntgendichten Markierungsrings. Dieser blockt Röntgenstrahlen ab und ermöglicht wie andere Markierungen das Erkennen der exakten Position des Ballonkatheters im betreffenden Organ. Dies ist wichtig, um das korrekte Einführen/Hinausziehen des Ballonkatheters im betreffenden Organ zu verfolgen. Die erwähnten Markierungen sind beim Einführen eines Katheters, aber auch bei Dauerkathetern wichtig. Dauerkatheter verbleiben über einen längeren Zeitraum im Menschen/Tier, deren Position muss periodisch überprüft werden.

In Bezug auf das Verfahren zum Betrieb des Ballonkatheters mit wenigstens zwei separaten, parallel längslaufenden Ausdehnungsräumen wird die Aufgabe erfindungsgemäss dadurch gelöst, dass die Ausdehnungsräume mit dem gleichen oder unterschiedlichen Arbeitsdrücken beaufschlagt werden.

Speziell ausgebildete Ballonkatheter weisen im Balloninneren einen oder mehrere befüllbare Ausdehnungsräume und/oder zwischen zwei Ballonhüllen einen oder mehrere befüllbare Ausdehnungsräume auf, insgesamt aber wenigstens deren zwei. Die einzelnen Ausdehnungsräume können mit dem gleichen oder mit verschiedenem Druck beaufschlagt werden, insbesondere wird in einem zylinderförmigen Innenraum ein niedrigerer Arbeitsdruck als in einem mantelförmigen äusseren Ausdehnungsraum aufrecht erhalten.

Ein erfindungsgemässer Ballonkatheter wird beispielsweise hergestellt, indem zwei oder mehr Ballonhüllen unabhängig voneinander ineinander gezogen und an wenigstens einer Stelle miteinander, mit dem Führungsdraht, mit dem Führungsdrahtlumen verschweisst und/oder verklebt werden, allenfalls mit einem weiteren Rohr, z. B. einem Blutzirkulationsrohr oder einem Lumen zu Diagnosezwecken.

Dieses Herstellverfahren erlaubt beispielsweise
- eine individuelle Materialauswahl und Materialausgestaltung der Ballonhüllen,
- ein individuelles Beschichten und/oder Anbringen von Farben/Markierungen,
- ein individuelles Platzieren des oder der Lumen/Rohre,
- ein individuelles Abdichten und/oder
- ein Testen des Ballonkatheters bezüglich der Funktionalität einzelner Ballonhüllen/Elemente.

Der erfindungsgemässe Ballonkatheter stellt im Vergleich zum Stand der Technik eine wesentliche Weiterentwicklung dar und ist als wegweisende Innovation im Dienste der Medizin für Mensch und Tier zu sehen.

Die Erfindung wird anhand von in der Zeichnung dargestellten Ausführungsbeispielen näher erläutert, welche auch Gegenstand von abhängigen Ansprüchen sind. Es zeigen schematisch:
- Fig. 1 einen Ballonkatheter mit einem Ausdehnungsraum in Doppelwandausführung,
- Fig. 2 einen Ballonkatheter gemäss Fig. 1 mit einem zusätzlichen Blutzirkulationsrohr, und
- Fig. 3 einen Ballonkatheter mit zwei Ausdehnungsräumen.

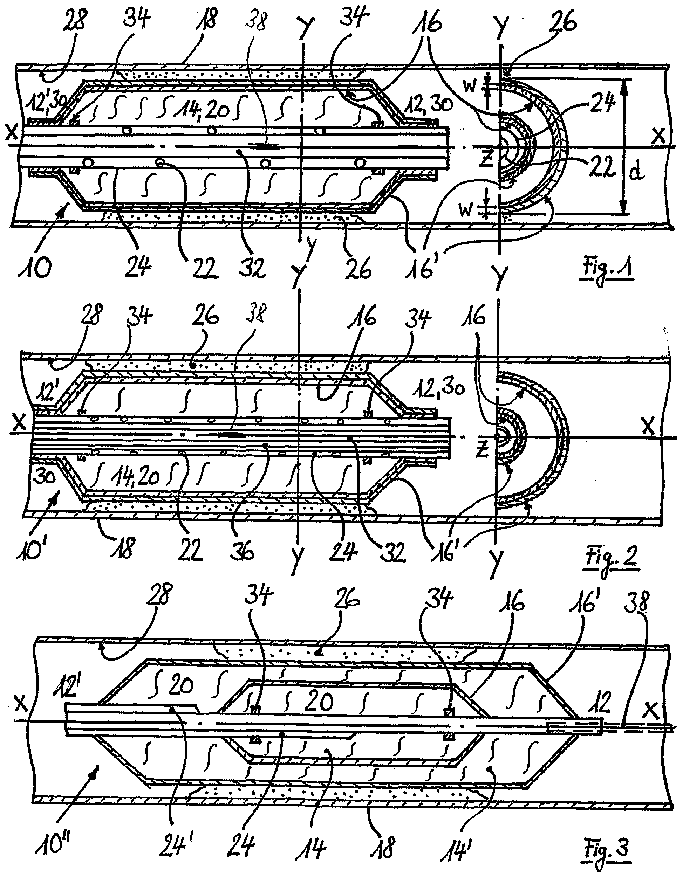

Fig. 1 stellt eine bevorzugte Ausführungsvariante des Ballonkatheters 10 mit einer Vorder- 12 und einer Rückseite 12' und einem einzelnen, inneren Ausdehnungsraum 14 entlang der Ballonkatheter-Längsachse x-x dar. Der Ballonkatheter 10 ist doppelwandig ausgeführt, wobei die Flächen zwischen der inneren (16) und der äusseren Ballonhülle (16') gleitfähig ineinander liegen. Die Ballonhüllen (16, 16') sind zur besseren Veranschaulichung auch im Querschnitt (y-y) dargestellt. Im Querschnitt (y-y) ist ausserdem die mit etwa 3 µm ausgesprochen dünne Wandstärke (w) einer einzelnen Ballonhülle 16 oder 16' und der Durchmesser d des Ballonkatheters unter maximalem Arbeitsdruck von etwa 45 bar eingezeichnet. Der Durchmesser d bei maximalem Arbeitsdruck weitet sich nur um etwa um 0,3 mm.

Der Ballonkatheter 10 ist in ein Hohlorgan 18, vorliegend in eine Blutbahn beziehungsweise ein Gefäss, eingeführt. Die Flüssigkeit in den Ballonhüllen 16, 16' wird über mehrere, vorteilhaft regelmässig verteilte Öffnungen 22 über ein nicht dargestelltes Anschlussstück und über ein Lumen 24 in den inneren Ausdehnungsraum 14 gepumpt beziehungsweise zum Entleeren des Ballonkatheters 10 von dort abgesaugt. Unter Arbeitsdruck wird der Ballonkatheter 10 ein blutbahnverengendes Gerinnsel 26 an die innere Gefässwand 28 gedrückt und der Ballonkatheter 10 gleichzeitig fixiert, worauf eine Diagnose und eine Therapie über entsprechende weitere Lumen 24 erfolgen können.

Die Ballonhüllen 16, 16' sind mit den Lumen 24 an den Stellen 30 der Vorder- und Rückseite 12, 12' verschweisst. Angedeutet ist auch ein Führungsdrahtlumen 32 um das Zentrum z mit einem nur teilweise eingezeichneten Führungsdraht.

Der in Fig. 1 dargestellte Ballonkatheter 10 wird nur kurzzeitig genutzt, wobei das Gefäss 18 durch den hohem Arbeitsdruck definiert aufgeweitet wird. Nach einer gewissen, durch den behandelnden Arzt festgelegten Zeit, wird die Flüssigkeit 20 aus dem Gefäss 18 abgesaugt, worauf sich der Ballonkatheter 10 entspannt. Das Gerinnsel 26 entspannt sich in der Regel zwar ebenfalls etwas, erreicht aber das ursprüngliche Volumen bei weitem nicht mehr, so dass die Blutzirkulation im Gefäss 18 nach dem Entfernen des Ballonkatheters 10 wieder wesentlich besser möglich ist.

Zwei röntgendichte Markierungsringe 34 ermöglichen das Lokalisieren des Ballonkatheters 10 mittels Röntgenstrahlen.

Fig. 2 zeigt einen Ballonkatheter 10' gemäss Fig. 1, der zusätzlich ein Blutzirkulationsrohr 36 entlang der Längsachse x-x, um das Zentrum z mit dem Führungsdraht 38 beziehungsweise und dem Führungsdrahtlumen 32, aufweist. Während Lumen 24 grundsätzlich via Rückseite 12' über das betreffende Hohlorgan 18 ins Freie führen, führt das Blutzirkulationsrohr 36 nicht wesentlich über ein Hohlorgan hinaus und ist vergleichsweise kurz, in der Regel etwa gleich lang wie ein Ballonkatheter 10'. Das Blutzirkulationsrohr 36 endet an den Schweissstellen 30 der Vorder- und der Rückseite 12, 12' des Ballonkatheters 10'.

Der Ballonkatheter 10' ist als Dauerkatheter ausgebildet und bleibt z.B. mehrere Tage eingesetzt. Dabei ermöglicht das Blutzirkulationsrohr 36 den freien Durchfluss des Blutes in Längsrichtung x-x. Die röntgendichten Markierungsringe 34 ermöglichen das periodische Lokalisieren des Ballonkatheters.

Fig. 3 zeigt einen doppelwandigen Ballonkatheter 10" mit zwei getrennten Ausdehnungsräumen, einen kürzeren inneren 14 und einen längeren äusseren 14'. Die beiden Ausdehnungsräume 14, 14' werden separat über jeweils ein Lumen 24, 24' gespeist. Der Ballonkatheter 10" ist in ein Hohlorgan 18 eingeführt, mit Flüssigkeit 20 gefüllt und steht unter Arbeitsdruck. Wie in Fig. 1 wird ein Gerinnsel 26 so stark an die Gefässwand 28 gepresst.

Die beiden getrennten Ausdehnungsräume 14, 14' können mit gleichen oder unterschiedlichen Arbeitsdrücken beaufschlagt werden. Je grösser ein Arbeitsdruck im äusseren Ausdehnungsraum 14' relativ zum inneren 14 ist, desto kleiner ist das Volumen des inneren Ausdehnungsraums 14 im Vergleich zum äusseren 14'. Im Extremfall kann auch nur einer der beiden Ausdehnungsräume 14, 14' mit Druck beaufschlagt werden, wodurch sich nur der entsprechende Ausdehnungsraum 14 oder 14' bildet.

In Fig. 3 ist der Führungsdraht 38 gestrichelt angedeutet, der aber nur an der Vorderseite 12 des Führungsdrahtlumens 32 eingesetzt wird und nicht durchgehend wie in Fig. 1 und 2 ist.

In einer nicht dargestellten Ausführungsvariante ist der Ballonkatheter 10" gemäss Fig. 3 entlang der Längsachse x-x um das Zentrum z mit einem Blutzirkulationsrohr 36 und/oder mit einem Diagnoselumen versehen.

In ebenfalls nicht dargestellten Ausführungsvarianten schliesslich erfolgt eine Zweiteilung der Ausdehungsräume etwa rechtwinklig zur Längsachse (x-x) oder entlang der Achse (x-x), ausgeführt mit oder ohne Diagnoselumen beziehungsweise mit oder ohne Bluzirkulationsrohr (36) im Zentrum (z). In beiden Varianten werden jeweils zwei innere Ballonhüllen (16) und eine äussere Ballonhülle (16') eingesetzt. Die beiden inneren Ballonhüllen (16) sind physisch voneinander getrennt. Sie werden über jeweils ein Lumen (24) von der selben Quelle mit dem selben Arbeitsdruck oder unabhängig voneinander von zwei verschiedenen Quellen mit unterschiedlichen Arbeitsdrücken beaufschlagt. In beiden Varianten liegen die äusseren Ballonhüllen (16') auf den inneren (16) und sind übereinander gleitfähig ausgebildet.

## Patentansprüche

1. Ballonkatheter (10, 10', 10") zur Einführung in ein im Wesentlichen röhrenförmiges Hohlorgan (18) im menschlichen oder tierischen Körper mit
- wenigstens einem befüll-und entleerfaaren Ausdehnungsraum (14, 14') zwischen einer Vorder- und einer Rückseite (12, 12'), welchem Ausdehnungsraum (14, 14') über wenigstens ein Anschlussstück mit wenigstens einem Lumen (24) ein flüssiges oder gasförmiges Medium (20) zu- und abführbar ist, und
- einem Führungsdrahtlumen (32) mit einem flexiblen Führungsdraht (38) entlang einer Ballonkatheter-Längsachse (x-x), wobei
- der Ballonkatheter (10, 10', 10") wenigstens zwei ineinander angeordnete Ballonhülle (16, 16') aufweist, welche gleitfähig übereinander gelegt sind, und
**dadurch gekennzeichnet, dass**
zwischen den wenigstens zwei Ballonhüllen wenigstens eine der direkt aufeinander liegenden Ballonhüllen mit einer dünnen Beschichtung versehen ist, welche die Gleitfähigkeit erhöht.

2. Ballonkatheter (10, 10', 10") nach Anspruch 1, **dadurch gekennzeichnet, dass** er in Doppelwandausführung mit direkt aufeinander liegenden Ballonhüllen (16, 16') für einen maximalen Arbeitsdruck von etwa 50 bar, vorzugsweise 40 bis 45 bar, ausgelegt ist, und sich bei maximalem Arbeitsdruck im Durchmesser (d) maximal etwa 0,3 mm aufweitet.

3. Ballonkatheters (10, 10', 10") nach Anspruch 1, **dadurch gekennzeichnet, dass** er in Dreifachwandausführung mit direkt aufeinander liegenden Ballonhüllen (16, 16') für einen maximalen Arbeitsdruck von etwa 60 bar, vorzugsweise 50 bis 55 bar, ausgelegt ist.

4. Ballonkatheter (10, 10', 10") nach einem der Ansprüche 1 bis 3, **dadurch gekennzeichnet, dass** die Ballonhüllen (16, 16') aus einem flexiblen, aber nur geringfügig dehnbaren Werkstoff bestehen, sich bei maximalem Arbeitsdruck im Durchmesser (d) maximal etwa 0,2 mm aufweitet, vorzugsweise aus einem Polyamid, Nylon und/oder Polyester.

5. Ballonkatheter (10, 10', 10") nach einem der Ansprüche 1 bis 4, **dadurch gekennzeichnet, dass** eine einzelne Ballonhülle (16, 16') eine Wandstärke (w) von 1-10 µm, vorzugsweise etwa 3 µm, aufweist.

6. Ballonkatheters (10, 10', 10") nach einem der Ansprüche 1 bis 5, **dadurch gekennzeichnet, dass** er mehrere befüllbare Ausdehnungsräume (14, 14') im Balloninneren und/oder mehrere befüllbare Ausdehnungsräume (14, 14') zwischen zwei Ballonhüllen (16, 16') aufweist.

7. Ballonkatheter (10, 10', 10") nach einem der Ansprüche 1 bis 6, **dadurch gekennzeichnet, dass** wenigstens eine Ballonhülle (16, 16') und/oder der Führungsdraht (38) wenigstens teilweise gefärbt, reflektierend und/oder fluoreszierend ausgebildet ist.

## Claims

1. Balloon catheter (10, 10', 10'') for insertion into a substantially tubular hollow organ (18) in the human or animal body, with
- at least one fillable and emptiable expansion space (14, 14') between a front and a rear (12, 12'), a liquid or gaseous medium (20) being able to be conveyed to and from the expansion space (14, 14') via at least one attachment piece with at least one lumen (24), and
- a guide wire lumen (32) with a flexible guide wire (38) along a longitudinal axis (x-x) of the balloon catheter, wherein
- the balloon catheter (10, 10', 10'') has at least two balloon sleeves (16, 16'), which are arranged one inside the other and are laid slidably over each other, and
**characterized in that**, between the at least two balloon sleeves, at least one of the balloon sleeves lying directly on each other is provided with a thin coating that increases the slidability.

2. Balloon catheter (10, 10', 10'') according to Claim 1, **characterized in that** it is designed, in a double-wall configuration with balloon sleeves (16, 16') lying directly on each other, for a maximum working pressure of approximately 50 bar, preferably 40 to 45 bar and, at the maximum working pressure, widens in diameter (d) to a maximum of about 0.3 mm.

3. Balloon catheter (10, 10', 10'') according to Claim 1, **characterized in that** it is designed, in a triple-wall configuration with balloon sleeves (16, 16') lying directly on each other, for a maximum working pressure of approximately 60 bar, preferably 50 to 55 bar.

4. Balloon catheter (10, 10', 10'') according to one of Claims 1 to 3, **characterized in that** the balloon sleeves (16, 16') are made from a flexible but only slightly extensible material, widening in diameter (d) by a maximum of about 0.2 mm at the maximum working pressure, preferably from a polyamide, nylon and/or polyester.

5. Balloon catheter (10, 10', 10'') according to one of Claims 1 to 4, **characterized in that** an individual balloon sleeve (16, 16') has a wall thickness (w) of 1-10 µm, preferably about 3 µm.

6. Balloon catheter (10, 10', 10'') according to one of Claims 1 to 5, **characterized in that** it has several fillable expansion spaces (14, 14') in the balloon interior and/or several fillable expansion spaces (14, 14') between two balloon sleeves (16, 16').

7. Balloon catheter (10, 10', 10'') according to one of Claims 1 to 6, **characterized in that** at least one balloon sleeve (16, 16') and/or the guide wire (38) are at least partially dyed, reflecting and/or fluorescent.

## Revendications

1. Cathéter à ballonnet (10, 10', 10"), pour introduction dans un organe creux (18) pour l'essentiel tubulaire dans le corps humain ou animal, comprenant
- au moins une chambre de dilatation (14, 14'), pouvant être remplie et vidée, entre une face avant et une face arrière (12, 12'), un fluide liquide ou gazeux (20) pouvant, par au moins une pièce de raccordement comportant au moins une lumière (24), être amené dans la chambre de dilatation (14, 14') et pouvant en être évacué, et
- une lumière (32) pour fil-guide, comprenant un fil-guide (38) flexible le long d'un axe longitudinal (x-x) du cathéter à ballonnet,
- le cathéter à ballonnet (10, 10', 10") comprenant au moins deux gaines de ballonnet (16, 16'), disposées l'une dans l'autre, qui sont superposées l'une à l'autre avec possibilité de glissement, et
**caractérisé en ce que**, entre les au moins deux gaines de ballonnet, au moins l'une des gaines de ballonnet directement superposées l'une à l'autre est pourvue d'un revêtement mince, qui augmente l'aptitude au glissement.

2. Cathéter à ballonnet (10, 10', 10") selon la revendication 1, **caractérisé en ce qu'**il est conçu selon une configuration en double paroi, avec des gaines de ballonnet (16, 16') directement superposées l'une à l'autre, pour une pression de travail maximale d'environ 50 bar, de préférence de 40 à 45 bar, et que, à la pression de travail maximale, son diamètre (d) s'élargit au maximum d'environ 0,3 mm.

3. Cathéter à ballonnet (10, 10', 10") selon la revendication 1, **caractérisé en ce qu'**il est conçu selon une configuration à triple paroi, avec des gaines de ballonnet (16, 16') directement superposées l'une à l'autre, pour une pression de travail maximale d'environ 60 bar, de préférence de 50 à 55 bar.

4. Cathéter à ballonnet (10, 10', 10") selon l'une des revendications 1 à 3, **caractérisé en ce que** les gaines de ballonnet (16, 16') sont constituées d'un matériau souple, mais qui n'est que peu dilatable, dont le diamètre (d) s'élargit au maximum d'environ 0,2 mm sous la pression de travail maximale, de préférence en un polyamide, un nylon et/ou un polyester.

5. Cathéter à ballonnet (10, 10', 10") selon l'une des revendications 1 à 4, **caractérisé en ce qu'**une gaine de ballonnet individuelle (16, 16') présente une épaisseur de paroi (w) de 1 à 10 µm, de préférence d'environ 3 µm.

6. Cathéter à ballonnet (10, 10', 10") selon l'une des revendications 1 à 5, **caractérisé en ce qu'**il comprend dans l'intérieur du ballonnet plusieurs chambres de dilatation (14, 14') pouvant être remplies et/ou entre deux gaines de ballonnet (16, 16') plusieurs chambres de dilatation (14, 14') pouvant être remplies.

7. Cathéter à ballonnet (10, 10', 10") selon l'une des revendications 1 à 6, **caractérisé en ce qu'**au moins une gaine de ballonnet (16, 16') et/ou le fil-guide (38) sont conçus au moins partiellement colorés, réfléchissants et/ou fluorescents.
